**Europäisches Patentamt**

(19) **European Patent Office**

**Office européen des brevets**

(11) **EP 0 781 844 A1**

(12) **EUROPEAN PATENT APPLICATION**
published in accordance with Art. 158(3) EPC

(43) Date of publication:
02.07.1997 Bulletin 1997/27

(21) Application number: 95934284.1

(22) Date of filing: 12.10.1995

(51) Int. Cl.$^6$: **C12N 15/12**, C12N 7/01, A61K 35/76, A61K 48/00, C12Q 1/68, G01N 33/563, C07K 16/18

(86) International application number:
PCT/JP95/02090

(87) International publication number:
WO 96/12017 (25.04.1996 Gazette 1996/18)

(84) Designated Contracting States:
AT BE CH DE DK ES FR GB GR IE IT LI LU MC NL PT SE

(30) Priority: 14.10.1994 JP 249226/94

(71) Applicants:
• OTSUKA PHARMACEUTICAL CO., LTD.
Chiyoda-ku Tokyo 101 (JP)
• THE AUSTRALIAN NATIONAL UNIVERSITY
Acton, ACT 0200 (AU)

(72) Inventors:
• SHINDO, Yutaka
Itano-gun, Tokushima 771-02 (JP)

• NISHIDA, Tsutomu
Naruto-shi, Tokushima 772 (JP)
• ADACHI, Masakazu
Takasaki-shi, Gunma 370 (JP)
• NAORA, Hiroto
Canberra, ACT 2611 (AU)
• NAORA, Honami
Canberra, ACT 2611 (AU)

(74) Representative: Hansen, Bernd, Dr.Dipl.-Chem. et al
Hoffmann, Eitle & Partner
Patentanwälte
Postfach 81 04 20
81904 München (DE)

(54) **GENE PARTICIPATING IN APOPTOSIS**

(57) The invention provides an apoptosis-associated gene comprising a nucleotide sequence coding for the amino acid sequence of SEQ ID NO:1. This gene can be utilized for the expression and detection of an apoptosis-associated protein and is useful for the diagnosis and pathological elucidation of various apoptosis-associated diseases or the prophylaxis and therapy of apoptosis-associated diseases through induction or inhibition of apoptosis.

EP 0 781 844 A1

**Description**

FIELD OF THE INVENTION

The present invention relates to an apoptosis-associated gene and, more particularly, to a novel nbl gene expressed at a high level in an early stage of apoptosis. The invention further relates to a method of detecting apoptosis-associated gene expression by use of the specific apoptosis gene, a method of controlling apoptosis, and the use of antisense sequences of the apoptosis-associated gene to control apoptosis.

PRIOR ART AND PROBLEMS

Apoptosis is defined as a process leading cells to death as observed in cell death either programmed in the development, differentiation and maturation of cells or induced under various conditions [Br. J. Cancer, 265, p. 239 (1972)] and taking place under various physiological conditions. Morphologically, apoptosis is characterized by separation of cells from surrounding cells, cytoplasmic shrinkage, chromatin condensation and pyknosis which may be associated with enhanced endonuclease activities, and nuclear fragmentation. Furthermore, disappearance of cell surface microvilli, membrane blebbing and other phenomena are also observed. Due to the enhanced endonuclease activity, DNA fragmentation is also observed and phagocytosis of apoptotic bodies, which are final fragments of cells, by neighboring cells has been discussed as a mechanism involved therein [Immunology Today, 7 (4), 115-119 (1986); Science, 245, 301-305 (1989)].

It has already been shown that apoptosis is involved, and plays important roles, in a variety of diseases. In recent years, therefore, particular attention has been paid to the potential value for the diagnosis of such diseases and also for the prevention and treatment of the diseases by inducing or inhibiting apoptosis.

Among those diseases in which induction of apoptosis is desirable are cancers such as leukemia and melanoma, autoimmunity, and viral diseases not associated with cell death. The diseases in which suppression of apoptosis is desirable include neuropathy caused by death of nerve cells, such as Alzheimer's disease, HIV infection (asymptomatic phase), and viral infections accompanied by cell death.

If an apoptosis-associated gene capable of controlling cell apoptosis through expression of the gene or the gene product could be provided, it would become possible to clarify the pathological aspects of the above-mentioned apoptosis or associated diseases and to establish some or other methods of diagnosing and treating said diseases based on the findings obtained by analyzing the expression levels of the said gene in various cells and the structure and function thereof or analyzing the product of expression thereof.

However, it is currently difficult to make such detailed analytical investigations concerning apoptosis, since apoptosis-associated genes have not yet been studied in detail.

DISCLOSURE OF INVENTION

In accordance with the present invention, involvement of a specific gene in the process of apoptosis is revealed. It has now been found that DNA fragmentation, a hallmark of apoptosis, is observed after temporal expression of such a gene and that the transient enhancement and the subsequent halt of expression are involved in the process of apoptosis.

Accordingly, the present invention provides such a specific apoptosis-associated gene. The present invention also relates to the use of the said gene for the diagnosis of various apoptosis-associated diseases and bio-medical studies of such diseases and furthermore for induction or suppression of apoptosis, through detection of the said gene or the corresponding expression product or artificial menipulation thereof.

The present invention provides a specific gene (hereinafter referred to as nbl gene), among apoptosis-associated genes, which comprises of a nucleotide sequence coding for the amino acid sequence of SEQ ID NO:1.

The present invention further provides the following nbl homologs as apoptosis-associated genes as well: fte-1 gene, RPS3a gene, MFT-1 gene, cyc07 gene, TU-11 gene, and KRP-A gene, all of which are highly homologous with the nbl gene specified by the nucleotide sequence coding for the amino acid sequence of the above SEQ ID NO:1.

Hereinafter, in the present specification, amino acids, peptides, nucleotide sequences, nucleic acids and so forth are expressed in terms of abbreviations according to the IUPAC-IUB rules, "Guidelines for preparing specifications etc. containing nucleotide sequences or amino acid sequences" (edited by the Japanese Patent Office) and common practice in the relevant fields of research.

The gene provided by the present invention is characterized by changes in its expression in the process of apoptosis. A specific example of such gene has the nucleotide sequence shown in SEQ ID NO:2, which contains the nucleotide sequence of the cDNA clone shown in SEQ ID NO:3, and this clone possesses an open reading frame composed of 792 nucleotides coding for 264 amino acid residues.

In SEQ ID NO:2 and SEO ID NO:3, the gene of the present invention is shown in a single-stranded DNA sequence.

However, the present invention also includes a DNA sequence complementary to such a single-stranded DNA sequence and thus emcompasses a component comprising both of these. The sequence of the gene of the present invention, shown in SEQ ID NO:2, is merely an example of the possible combination of codons for the respective amino acid residues. It is a matter of course that the gene of the present invention can have not only the said sequence but also a DNA sequence in which, where possible, an arbitrary codon is selected for each amino acid residue for the purpose of combination. The said codon selection can be made in the conventional manner, for example based on the codon frequency pattern in the host employed [Nucl. Acids Res., 9, 43-74 (1981)].

Furthermore, the gene of the present invention includes those DNA sequences that code for equivalent polypeptides having the same functions as those of the original gene. Such modification can be made by substitution, deletion and/or addition of partial amino acid and/or amino acid sequence shown in SEQ ID NO:1. While production, modification (mutation) or the like of these polypeptides may occur spontaneously, they can be produced by posttranslational modification or by modifying a native gene (the gene of the present invention) by a genetic engineering technique, for example site-specific mutagenesis [Methods in Enzymology, 154, p. 350, 367-382 (1987); ibid., 100, p. 468 (1983); Nucleic Acids Research, 12, p. 9441 (1984); Zoku Seikagaku Jikken Koza 1, "Idenshi Kenkyuho (Methods for the Study of Genes) II", edited by the Japanese Biochemical Society, p. 105 (1986)] or by synthesizing a mutant DNA by means of chemical synthesis using the phosphotriester method or phosphoamidite method [J. Am. Chem. Soc., 89, p 4801 (1967); ibid., 91, p. 3350 (1969); Science, 150, p. 178 (1968); Tetrahedron Lett., 22 p. 1859 (1981); ibid., 24, p. 245 (1983)] or the like, or by using a combination of these techniques.

In fact, the human fte-1 gene (GenBank Accession No. M84711) corresponding to the rat fte-1 gene [Proc. Natl. Acad. Sci. USA 89, 2200-2204 (1992)], also known as a v-fos transformation effector gene, contains (A) at the 620th nucleotide in the nucleotide sequence of SEQ ID NO:3 and thus the corresponding 196th amino acid is Asp, giving an equivalent in activity. Accordingly, the said gene is included within the scope of the apoptosis-associated gene of the present invention.

Human RPS3a [Metspalu, A., et al., Gene, 119, 313-316 (1992)] gene which encodes a ribosome protein differs from nbl gene only in the 196th amino acid residue and, therefore, falls within the scope of the apoptosis-associated gene of the present invention.

Moreover, the homology of yeast MFT-1 gene, which is associated with the transport of proteins to mitochondria, with nbl gene is 58% at the amino acid level [Garrett, J. M., et al., Mol. Gen. Genet., 225, 483-491 (1991)] and, therefore, this gene also falls within the scope of the apoptosis-associated gene according to the present invention.

Furthermore, nbl gene shows a homology of 62% at the amino acid level with cyc07 gene which modulates (controls) the cell cycle of higher plants [Ito, M., et al., FEBS Letters, 301, 29-33 (1992)] and, therefore, this cyc07 gene also falls within the scope of the apoptosis-associated gene according to the present invention.

In addition, mouse TU-11 gene [Gordon, H. M., et al., J. Immunol., 148, 4021-4027 (1992)] differing from nbl gene only in 4 amino residues and showing a homology of 98% with the latter at the amino acid level and sea hare KRP-A [Sea hare; Aplysia californica; Auclair, D., et al., Eur. J. Biochem., 220, 997-1003 (1994)] gene showing a homology of 78% with nbl gene at the amino acid level are also subsumed in the concept of apoptosis-associated gene of the present invention.

The above apoptosis-associated protein can be produced in a stable manner by utilizing the gene of the present invention, for example inserting it in a microorganism vector and cultivating the transformed microorganism.

The apoptosis-associated protein obtained by utilizing the gene of the present invention can be used also in producing a specific antibody. The component to be used here as the antigen may be the protein produced in large amounts using the above-mentioned genetic engineering techniques and the antibody obtained may be either a polyclonal one or a monoclonal one. These antibodies can advantageously be used in purifying, assaying or identifying the apoptosis-associated protein, for instance.

In particular, the gene of the present invention is characterized by the fact that a part or the whole of the nucleotide sequence thereof can be very favorably used in detecting the expression of the apoptosis-associated gene in various human tissues or cells.

The gene of the present invention can be readily produced by using conventional genetic engineering techniques [Molecular Cloning, 2nd ed., Cold Spring Harbor Laboratory Press (1989); Zoku Seikagaku Jikken Koza "Idenshi Kenkyuho I, II and III", edited by the Japanese Biochemical Society (1986) and elsewhere] based on the sequence information about the gene of the present invention as disclosed herein.

This can be achieved, for example, by selecting a desired clone from a human cDNA library (prepared in the conventional manner using appropriate source cells in which the apoptosis-associated gene is expressed) using an appropriate probe or probes or antibody specific to the gene of the present invention [Proc. Natl. Acad. Sci. USA 78, 6613 (1981); Science, 222, 778 (1983) and elsewhere].

In the procedure mentioned above, the source cells include various cells, tissues or cultured cells derived from these, for instance, in which the apoptosis-associated gene is expressed. Isolation of total RNA from these sources, isolation and purification of mRNA, conversion to (synthesis of) cDNA and cloning thereof and other steps can be carried out in the conventional manner. Commercial cDNA libraries are also available. Those cDNA libraries, for example various cDNA libraries commercially available from Clontech Lab. Inc., can also be used.

Screening of the gene of the present invention from these cDNA libraries can be performed by the conventional method mentioned above. These screening methods, include, for example, the method comprising the detection of the proteins produced by cDNA clones by immunological assays using a specific antibody to the apoptosis-associated protein, the plaque or colony hybridization method using probes selectively binding to the desired DNA sequence, and a combination of these. The probes to be used here are generally DNA sequences chemically synthesized on the basis of the information about the DNA sequence of the gene of the present invention, among others. The gene itself of the present invention or fragments thereof may of course be used as the probes.

Further, the DNA/RNA amplification method using the PCR technique [Science, 230, 1350-1354 (1985)] can advantageously be used for obtaining the gene of the present invention. Particularly when the full-length cDNA is difficult to obtain from the library, the RACE method (RACE: Rapid amplification of cDNA ends; Jikken Igaku (Experimental Medicine), 12 (6), 35-38 (1994)] can preferentially be employed. The primers to be used in such PCR method can be suitably selected based on the sequence information about the gene of the present invention as disclosed herein and can be synthesized by a conventional method.

The amplified DNA/RNA fragment can be isolated and purified in the conventional manner, as mentioned above, for example by gel electrophoresis.

Nucleotide sequence determination of the gene of the present invention obtained as mentioned above or of various DNA fragments or the like can be carried out in the conventional manner, for example by the dideoxy method [Proc. Natl. Acad. Sci. USA 74, 5463-5467 (1977)] or the Maxam-Gilbert method [Methods in Enzymology, 65, 499 (1980)]. Such nucleotide sequence determination may also be conducted with ease using a commercial sequencing kit or the like.

By using the gene of the present invention, a recombinant apoptosis-associated protein can be obtained following the general recombinant DNA technology [refer to e.g. Science, 224, p. 1431 (1984); Biochem. Biophys. Res. Comm., 130, p. 692 (1985); Proc. Natl. Acad. Sci. USA, 80, p. 5990 (1983) and the references cited above]. More particularly, the production of the said apoptosis-associated protein is carried out by preparing a recombinant DNA capable of being expressed in host cells, introducing this into the host cells for transformation and culturing the resulting transformant.

The host cells to be used here may be either eukaryotic cells or prokaryotic cells. The said eukaryotic cells include vertebrate cells, yeast cells and the like. The vertebrate cells include, but are not limited to, those in common use, such as COS cells [Cell, 23, 175-182 (1981)], which are simian cells, Chinese hamster ovary cells and a dihydrofolate reductase-deficient mutant thereof [Proc. Natl. Acad. Sci. USA 77, 4216-4220 (1980)].

The expression vector to be used in vertebrate cells may be one containing a promoter generally located upstream of the gene to be expressed, RNA splicing sites, a polyadenylation site, a transcription termination sequence, etc. and this may have a replication origin, as necessary. As examples of the said expression vector, there may be mentioned pSV2dhfr [Mol. Cell. Biol., 1, 854-764] having the SV40 early promoter and the like. Yeasts are also used generally and frequently as eukaryotic microorganisms and, among others, yeasts belonging to the genus Saccharomyces can advantageously be used. For example, as the expression vector in eukaryotic microorganisms such as yeasts, pAM82 [Proc. Natl. Acad. Sci. USA, 80, 1-5 (1983)] can be used. This vector contains a promotor for the acid phosphatase gene, and the like.

Generally and frequently used as the prokaryotic host are Escherichia coli and Bacillus subtilis. When any of these is used as the host in the practice of the present invention, an expression plasmid derived from a plasmid vector capable of replicating in the said host microorganisms can be used. This vector contains a promoter, the SD (Shine and Dalgarno) sequence upstream from the gene of the present invention and an initiation codon necessary for the initiation of protein synthesis (e.g. ATG) so that the said gene can be expressed in the host microorganisms. The strain Escherichia coli K12 and the like are often used as the host Escherichia coli, and pBR322 and modifications thereof are generally used as the vectors. These are, however, not exclusive choices. Other per se known various strains and vectors can be used as well. Usable as the promoter are, for example, the tryptophan (trp) promoter, 1pp promoter, lac promoter, PL/PR promoter, etc.

In order to transform host cells with the thus-obtained desired recombinant DNA, various general methods can be employed. The transformants obtained can be cultured in the conventional manner and the culture leads to production and expression of the desired apoptosis-associated protein encoded by the gene of the present invention. The medium to be used in the said culture can be selected from among various convential media depending on the host cells employed. The culture can be conducted under conditions suited for the growth of the host cells.

In the above manner, the desired recombinant apoptosis-associated protein is expressed, produced and accumulated, or secreted intracellularly, extracellularly or on the cell membrane.

The recombinant apoptosis-associated protein can be isolated and purified, as desired, by various separation procedures utilizing the physical, chemical and other properties thereof [refer to "Seikagaku (Biochemistry) Data Book II", pages 1175-1259, 1st edition, 1st printing, published June 23, 1980 by Tokyo Kagaku Dozin; Biochemistry, 25 (25), 8274-8277 (1986); Eur. J. Biochem., 163, 313-321 (1987) and elsewhere]. As examples of such methods, there may be mentioned, more particularly, conventional reconstitution treatment, treatment with a protein precipitating agent (salting out method), centrifugation, osmotic shock procedure, sonication, ultrafiltration, molecular sieve chromatography (gel filtration), adsorption chromatography, ion exchange chromatography, affinity chromatography, high-performance liquid chromatography (HPLC) and other various liquid chromatographic techniques, dialysis, and combinations of these.

The present invention reveals a specific gene involved in the process of apoptosis through the expression thereof and is based on contribution clarifying such novel function thereof.

Accordingly, the gene of the present invention has utility particularly in the fields of diagnosis and drugs.

For example, based on the sequence information about the gene of the present invention as revealed by the present invention, the expression of the apoptosis-associated gene in various human tissues can be detected using a part or the whole of the nucleotide sequence of the said gene. This can be successfully realized in the conventional manner, for example by RNA amplification by RT-PCR [Kawasaki, E. S., Amplification of RNA. In PCR Protocol, A guide to methods and applications, Academic Press, Inc., San Diego, 21-27 (1991)] or Northern blotting analysis [Molecular Cloning, Cold Spring Harbor Laboratory (1989)].

The morphological change of cells in the apoptosis induced in various cells can be confirmed by changes of cells such as protuberances on the cell surface, shrinkage and light refraction as observable by microscopic examination of trypanblue stained culture. On the other hand, the intracellular change in apoptosis can be confirmed by chromatin condensation around the nuclear membrane, nuclear fragmentation, etc. as observable on smear preparations which can be obtained by cytospin centifugation followed by other cell staining techniques.

Furthermore, both induction and inhibition of apoptosis can be confirmed by an analysis of DNA fragmentation generated by endonuclease activity. This analytical technique typically comprises homogenizing a tissue in a suitable buffer solution containing Tris-HCl, EDTA, SDS or the like, incubating the homogenate in the presence of proteinase K, extracting the resulting DNA with a suitable solvent. Then the DNA is subjected to agarose gel electrophoresis to confirm the presence of DNA fragmentation. Moreover, the amount of DNA fragmentation can be quantitated using a suitable quantitative analyzer.

In employing the PCR method, the primers to be used are not limited to any particular ones provided that they are specific to the gene of the present invention and can specifically amplify the gene of the present invention exclusively. Thus, they can suitably be selected based on the information disclosed herein. Generally, they can have a partial sequence of about 20 to 30 nucleotides in length, according to the conventional procedures.

Accordingly, the present invention also provides such primers and/or probes useful in detecting the apoptosis-associated gene.

Furthermore, apoptosis can be regulated by controlling the expression of the gene of the present invention or the activity of the corresponding gene product and such control can be utilized in the treatment of various diseases associated with apoptosis.

In cases where the expression of the gene (product) of the present invention is at a high level in tissues and/or cells involved in or related to various pathologic states or pathogenic factors, apoptosis can be induced by inhibiting the said gene. For instance, in a tumor in which the level of expression of the gene of the present invention is high, desired apoptosis can be induced in the said tumor by suppressing this expression or by inhibiting the activity of the gene product. On the other hand, in cases where apoptosis leads to a pathologic state or is a pathogenic factor and accordingly suppression of apoptosis means treatment of the pathologic state, apoptosis can be suppressed by preventing the decrease in the level of the gene (product) of the present invention by inducing or enhancing the expression of the gene of the present invention, or by maintaining the activity of the gene product.

As a more specific case of the above apoptosis induction, there can be mentioned the case which comprises enhancing the level of expression of the apoptosis-associated gene of the invention in a cell or tissue (the target cell) beyond a certain induction threshold level or up to a turning point at which the expression of the apoptosis-associated gene of the invention becomes maximal and then suppressing the expression level of the gene to a level below the threshold, whereby a potent induction of apoptosis can be obtained.

The factors for stimulating the apoptosis-associated gene expression in the target cell may be any agents that are capable of enhancing the expression of the objective gene and includes steroids among others.

As the factors for inhibiting the expression of the apoptosis-associated gene which is already at a high level, the antisense nucleotide sequences for the apoptosis-associated gene can be employed. A specific example is an nbl gene antisense oligonucleotide.

The induction threshold expression level of the objective apoptosis-associated gene can be individually selected according to the type of target cells but when the examples given hereinafter are taken as the reference, it is preferably not less than about 5-fold as high as the expression level of its mRNA in the nomal tissue cells. Moreover, the inhibition of the expression of the apoptosis-associated gene for the contemplated induction should be down to a level preferably below its mRNA level in the normal tissue.

For the purpose of inhibiting the above expression of the apoptosis-associated gene, the antibody to a protein expressed by the apoptosis-associated gene or a fragment of the antibody can be employed.

Such suppression and induction or enhancement of the expression of the gene of the present invention can be performed by any of the conventional methods. Since the target gene has now been identified, those skilled in the art could suitably select an appropriate method therefor. For gene suppression, for instance, the antisense nucleotide method, for example the method of inhibiting translation from mRNA [Science, 261, 1004-1012 (1993)] or the triple helix method for inhibiting transcription from DNA to RNA [Trends Biotech. 10, 132-136 (1992)] and the like can be employed as the means therefor. For induction or enhancement of expression of the gene of the present invention, gene transfer by means of a virus vector [Science, 260, 926-932 (1993)] or the like can be employed. For activity inhibition of the gene product, the use of a specific antibody as mentioned above may be advantageous.

The present invention thus further provides a method of controlling apoptosis which comprises artificially regulating the gene of the present invention.

In a further aspect, the present invention provides a therapeutic agent or composition and a therapeutic procedure for apoptosis-related diseases employing the apoptosis-associated gene antisense nucleotides or a protein expressed by the apoptosis-associated gene or an antibody to a protein expressed by the apoptosis-associated gene, or a fragment of the said antibody.

In accordance with the present invention, there is provided an apoptosis-associated gene and the use of the said gene makes it possible to detect the expression of the apoptosis-associated gene in various tissues, or to produce the apoptosis-associated protein using the genetic engineering technology, so that the said gene and protein can be employed, with good results, in diagnosing various apoptosis-associated diseases as well as in screening for and evaluating apoptosis inducers or inhibitors.

Furthermore, it is possible to induce or suppress apoptosis by controlling the expression of the gene of the present invention. This is useful in the prevention and treatment of various diseases associated with apoptosis.

BRIEF DESCRIPTION OF THE DRAWINGS

Fig. 1 is a graph showing the results obtained in Example 1-(5)-1) by investigating dexamethasone-induced apoptosis in terms of DNA fragmentation, nbl mRNA expression level and myc mRNA expression level.

Fig. 2 is a graph showing the relationship of apoptosis induction with the time-course of cell viability in the HL-60 cells treated with ActD in Example 1-(6)-4) of the present invention.

Fig. 3 is a graph showing the effect of ActD on cell viability after 6 and 24 hours of ActD treatment in various human cell lines as investigated in Example 1-(6)-5) of the present invention.

Fig. 4 is a graph showing the results obtained by investigating the relationship of the constitutive expression level of nbl mRNA with DNA fragmentation induced by ActD treatment in various human cell lines in accordance with Example 1-(6)-6) of the present invention.

Fig. 5 is a graph showing the results obtained by investigating the relationship of the expression level of nbl mRNA remaining at 6 hours after addition of ActD with DNA fragmentation in various human cell lines in accordance with Example 1-(6)-7) of the present invention.

Fig. 6 is a graph showing the results of comparison of nbl with myc in mRNA half-life in various human cell lines in accordance with Example 1-(6)-7) of the present invention.

Fig. 7 is a graph showing the results obtained by investigating the influence of nbl antisense oligomer on apoptosis and viability of HL-60 cells in accordance with Example 1-(6)-8).

Fig. 8 is a graph showing the results of an investigation of the effect of the incubation time and concentration dependency of nbl antisense oligomer on the apoptosis and viability of HL-60 cells in Example 1-(6)-8) of the present invention.

Fig. 9 is a graph showing the apoptotic morphology and DNA fragmentation induced by nbl antisense oligomer in HL-60 cells in Example 1-(6)-8) of the present invention.

BEST MODE FOR CARRYING OUT THE INVENTION

The following examples are further illustrative of the present invention.

Example 1

(1) Cloning of nbl cDNA:

1) Total RNA was extracted from Namalwa Burkitt lymphoma cells by the guanidinium-cesium chloride method and poly(A)+RNA was prepared by oligo(dT)-cellulose column chromatography (Molecular Cloning, pp. 196-198, Cold Spring Harbor Laboratory, 1982).

Double-stranded cDNA was prepared from the above poly(A)+RNA using reverse transcriptase and DNA polymerase I with oligo(dT) as a primer, and cDNA cloning was performed by the dC and dG tailing method (Molecular Cloning, pp. 239-242, Cold Spring Harbor Laboratory, 1982).

Namely, dC tailing was carried out at the 3'-end of the double-stranded cDNA using terminal deoxy-nucleotidyl transferase. Separately, the plasmid vector pBR322 was cleaved with the restriction enzyme PstI and dG tailing was conducted at the 3'-end thereof using terminal deoxynucleotidyl transferase. The thus-obtained dC-tailed cDNA and dG-tailed pBR322 were subjected to annealing and the annealing mixture was used to transform competent cells of Escherichia coli HB101 to give 1,700 tetracycline-resistant clones.

149 clones not less than 0.3 kb in cDNA length were randomly selected and cleaved with the restriction enzyme HinfI. Based on the cleavage fragment patterns, the most frequently found cDNA clones (8 clones out of 149 clones) were selected and named "nbl". Among the nbl clones, the one about 880 bp in cDNA length was named "Na880".

It was confirmed that the said clone Na880 has a sequence ranging from the 65th nucleotide to the 3'-end of the nucleotide sequence of SEQ ID NO:3. 2) Using 2 μg of the poly(A)+RNA mentioned above, 464 ng of cDNA was synthesized with oligo(dT) as a primer [cDNA synthesis system Plus (Amersham)].

A λgt10 cDNA library (205,000 pfu) was constructed from 37 ng of that cDNA using commercial kits [cDNA cloning system λgt10 adapter method (Amersham) and Gigapack II Gold (Stratagene)].

Using 110,000 clones of this library, plaques were produced with Escherichia coli NM514 as a host and immobilized on a Hybond N+ filter (Amersham), followed by prehybridization in a solution comprising 5 x SSC (SSC: 150 mM NaCl-15 mM sodium citrate (pH 7.0)], 10 x Denhardt's solution (Denhardt's solution: 0.02% bovine serum albumin-0.02% Ficoll-0.02% polyvinylpyrrolidone), 0.1% SDS and 100 μg/ml salmon sperm DNA at 65°C for 20 hours.

Further, hybridization was carried out in a solution comprising 5 x SSC, 10 x Denhardt's solution, 0.1% SDS, 100 μg/ml salmon sperm DNA and a probe at 65°C for about 20 hours.

The probe used was 40 ng of the Na880 DNA fragment labeled using a Multi primer label kit (Amersham).

After hybridization, the filter was rinsed two times in a solution comprising 2 x SSC and 0.1% SDS at room temperature for 15 minutes and further two times in a solution comprising 0.1 x SSC and 0.1% SDS at 65°C for 30 minutes.

After air drying, an X ray film (XAR-5; Kodak) was exposed to the filter. Each plaque region corresponding to the position of a positive signal on the film was scraped off and suspended in SM solution. Using this phage solution, plaque hybridization was again carried out under the same conditions as mentioned above.

In this manner, 38 positive clones were isolated. A portion of each of these 38 clones was incubated in a boiling water bath for 2 minutes and PCR was carried out using the λgt10 primer and the LA-PCR kit (Takara Shuzo) in a total of 30 cycles under the conditions: 94°C - 30 seconds, 55°C - 30 seconds and 72°C - 30 seconds (the extension time being 4 seconds in each cycle).

The PCR product was subjected to chloroform extraction and then to 1% agarose gel electrophoresis. The clone possessing the longest cDNA was selected, the inserted cDNA was subcloned in the EcoRI site of pUC118, and part of the nucleotide sequence of this inserted cDNA was determined using Sequenase DNA Sequence Kit (USB).

By confirming the nucleotide sequences of the 5'- and 3'-regions of the thus-obtained full-length clone "nbl-8" which correspond to Na880, the nucleotide sequence shown by SEQ ID NO:3 was obtained.

(2) Analysis of DNA fragmentation:

The tissue was homogenized in a lysis buffer [50 mM Tris-HCl (pH 8.0), 10 mM EDTA, 0.5% SDS] and then incubated overnight at 50°C in the presence of 200 μg/ml of proteinase K. After phenol and chloroform extraction, DNA was precipitated with ethanol and after removal of RNA with RNase A, subjected to analysis of DNA fragmentation.

10 μg of DNA was electrophoresed on 1% agarose gels containing 40 mM Tris-acetate (pH 7.8) and 1 mM EDTA. The extent of DNA fragmentation was quantified by laser densitometric analysis of negatives of gel photographs taken after electrophoresis.

(3) Northern blot analysis:

Total RNA was prepared by the one-step extraction method [Anal. Biochem., 162, 156-159 (1987)] comprising acidic guanidinium thiocyanate phenol chloroform extraction.

Namely, the tissue was homogenized with 4 M guanidinium isothiocyanate-25 mM sodium citrate-0.2 M sodium acetate (pH 4.0), 1 volume of phenol and 1/5 volume of chloroform-isoamyl alcohol (49:1) were added and the mixture was shaken and centrifuged. Isopropanol (1 volume) was added to the aqueous layer obtained by centrifugation and mixture was centrifuged to give an RNA pellet.

The RNA pellet obtained was washed with 80% ethanol, dried and dissolved in water.

Agarose electrophoresis of total RNA was carried out, using 20 μg of RNA, on 1.2% agarose gels containing 20 mM 3-(N-morpholino)propanesulfonic acid (pH 7.0)-5 mM sodium acetate-1 mM EDTA-2.2 M formaldehyde. After electrophoresis, RNA was transferred to a nitro-cellulose filter.

Hybridization was carried out overnight at 42°C in a solution comprising 50% formamide-25 mM $KH_2PO_4$ (pH 7.4)-

5 x SSC-5 x Denhardt's solution and 200 μg/ml salmon sperm DNA, using $^{32}$P-labeled DNA probes (about 2 x 10$^6$ cpm/ml).

The $^{32}$P-labeled DNA probes were prepared by the random primer method using [α-32$_{P]deoxyadenosinetri}$phosphate. The DNA probes used were as follows:

nbl probe: 0.9 kb PstI fragment of Na880;
myc probe: 1.3 kb ClaI-EcoRI fragment derived from the 9 kb human myc genomic DNA clone (Oncor Inc.);

After hybridization, the filter was rinsed in 1 x SSC-0.1% SDS at 65°C for 30 minutes and then in 0.1 x SSC-0.1% SDS at 65°C for 30 minutes. Then, autoradiography was performed at -70°C using a sensitized screen. Relative intensities of hybridization signals were quantified by laser densitometry and also by PhosphorImager analysis.

(4) Apoptosis and nbl gene expression in mouse tissues:

Various normal mouse tissues and mouse tumor tissues were examined for the extent of apoptosis by the DNA fragmentation analysis mentioned above.

Cerebellum, thymus, lung and liver tissues obtained from 4 week old mice (Balb/c, male) were used as normal tissues.

1-6 and 1-5 fibrosarcomas derived from mouse NIH 3T3 cells transformed with a mutated human c-H-ras gene as well as the lymphoma cell line AKR-2 were used as tumor tissues.

1-6 and 1-5 fibrosarcomas were inoculated subcutaneously into male nude mice. AKR-2 was inoculated intraperitoneally into AKR mice.

No DNA fragmentation was observed in normal tissues, whereas DNA fragmentation was observed in the fibrosarcoma 1-6 at the late tumor growth phase and, in the case of inoculation of the multiple nodules, also at the early growth phase. A lesser, yet clearly visible, degree of DNA fragmentation was observed in the fibrosarcoma 1-5. In AKR-2 as well, DNA fragmentation was observed.

Northern blot analysis described in (3) of nbl gene expression in the various tissues mentioned above revealed low levels of expression of the nbl gene in normal tissues (cerebellum, liver, thymus, lung). On the contrary, nbl mRNA expression was higher in fibrosarcoma 1-5 and AKR-2 and in small nodules of 1-6, in which apoptosis was induced, and elevated in 1-6 at the late growth phase.

In view of the above findings, it is considered that expression of the nbl gene is associated with the process of apoptosis although the relation between nbl gene expression and DNA fragmentation is not strictly proportional.

(5) Glucocorticoid-induced apoptosis and nbl gene expression:

1) Dexamethasone (250 μg) was dissolved in PBS and intraperitoneally administered to 4 week old male Balb/c mice. Before administration (0 hour) and after 3, 6, 9, 12, 18, 24 and 48 hours after injection, the thymus was excised and analyzed for DNA fragmentation using the procedure described above.

Northern blot analysis was also performed to examine nbl and myc mRNA expression using the procedure described above.

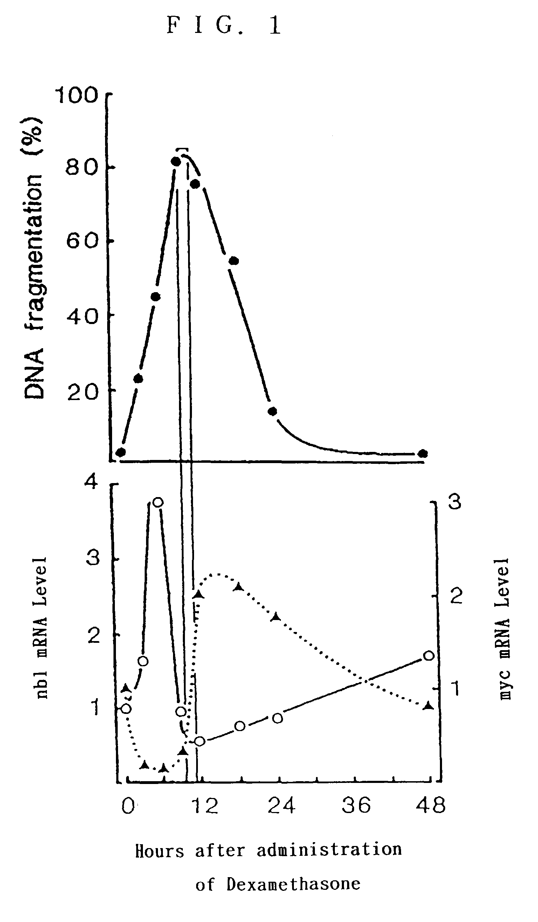

The results thus obtained are summarized in Fig. 1. In the figure, shaded circles indicate the extent of DNA fragmentation (%), open circles indicate nbl mRNA levels, and shaded triangles indicate myc mRNA levels.

DNA fragmentation occurred at a peak at 9 hours after dexamethasone administration, then declined and, after 24 hours, was hardly observable. Expression of nbl mRNA increased markedly after dexamethasone administration, reached a peak at 6 hours, then rapidly declined during the subsequent 3 hours and returned to the level observable in the untreated mouse thymus by 24 hours. Expression of myc mRNA decreased after dexamethasone administration and then (after 9 hours and thereafter) increased with the decrease of DNA fragmentation.

2) Actinomycin D administered in vivo is immediately taken up by specific organs such as kidney and excreted into urine. Consequently, the concentration of actinomycin D falls below the effective level soon after administration. This indicates that actinomycin D can be used as a short acting pulse inhibitor in vivo.

Therefore, actinomycin D was used to examine whether the above-mentioned temporal expression of nbl mRNA was associated with dexamethasone-induced thymic apoptosis or not.

Thus, actinomycin D was dissolved in water and administered intraperitoneally at a dose of 1.5 μg per gram of body weight 15 minutes before or 3 hours, 5.75 hours or 8 hours after dexamethasone administration. At 9 hours following dexamethasone administration, the thymus was excised and subjected to DNA fragmentation analysis.

Actinomycin D administration at 5.75 hours after dexamethasone administration resulted in strong inhibition of DNA fragmentation.

On the other hand, actinomycin D was administered simultaneously with dexamethasone and 5.5 hours after dexamethasone administration. At 6 hours after dexamethasone administration, the thymus was excised and ana-

lyzed for <u>nbl</u> mRNA expression.

Actinomycin D administration at 5.5 hours after dexamethasone administration inhibited <u>nbl</u> mRNA expression. Therefore, it was considered that the transient expression of the <u>nbl</u> gene is associated with <u>in vivo</u> thymic apoptosis induced by the glucocorticoid.

3) The above findings indicates the following.

Thus, in thymic apoptosis in mice as induced by administration of dexamethasone, which is a steroid agent, DNA fragmentation, a hallmark of apoptosis, is seen after temporal expression (transient rapid increase and the subsequent decrease) of the <u>nbl</u> gene. This result suggests that temporal involvement of the <u>nbl</u> gene (gene product) is required in the process leading to apoptosis.

In normal tissues, the level of expression of the <u>nbl</u> gene is low, whereas in many tumor tissues and tumor cells, the level of expression of the said gene is high. This suggests that tumor tissues and cells showing a high level of <u>nbl</u> gene expression are in a state in which the above-mentioned temporal <u>nbl</u> gene expression is at its peak in the process leading to apoptosis and that, the high expression of the said gene is being maintained, resulting in no apoptosis induction. Accordingly, it is anticipated that apoptosis may be induced in tumor tissues and cells by inhibiting the high expression of the <u>nbl</u> gene in tumor tissues and cells, as in the case of thymic apoptosis.

(6) The relation between apoptosis induction and constitutive <u>nbl</u> expression level in various cell lines:

1) Human tissue and cell culture

Promyelocytic leukemia cell line HL-60 [Collins, S. I., et al., Nature, <u>270</u>, 347-349 (1977)], histiocytic leukemia cell line U937 [Sundstrom, C., et al., Int. J. Cancer, <u>17</u>, 565-577 (1976)], and plasmacytoma cell line HS-Sultan [Harris, N. S., Nature, <u>250</u>, 507-509 (1974)] were respectively cultured in RPMI-1640 Medium (Gibco BRL; Gaithersburg, MD, U.S.A.) containing 10% FCS (fetal calf serum; CSL, Melbourne, Australia), 2 mM glutamine, 1 mM sodium pyruvate, 100 U/ml penicillin, and 100 $\mu$g/ml streptomycin at 37°C.

In addition, T-lymphoblastic leukemia cell line Jurkat [Gillis, S. and Watson, J., J. Exp. Med., <u>152</u>, 1709-1719 (1980)] was cultured in the above medium supplemented with 0.05 mM $\beta$-mercaptoethanol at 37°C.

Bladder cancer cell line 5637 [Welto, K. et al., Proc. Natl. Acad. Sci., USA., <u>82</u>, 1526-1530 (1985)] and liver cancer cell line HepG2 [Aden, D. P., et al., Nature, <u>282</u>, 615-616 (1979)] were respectively cultured in MEM (Gibco BRL; Gaithersburg, MD, U.S.A) supplemented with 20% FCS, 2 mM glutamine, 100 U/ml penicillin, 100 $\mu$g/ml streptomycin, and modified amino acids and modified vitamins (ICN, Sydney, Australia) at 37°C.

WISH amnionic cell line [Hayflick, L., Exp. Cell Res., <u>23</u>, 14 (1961)] and Chang liver cell line [Chang, R. S. Proc. Soc. Exp. Biol. Med., <u>87</u>, 440 (1954)] were cultured in MEM supplemented with 10% FCS, 2 mM glutamine, 100 U/ml penicillin, and 100 $\mu$g/ml streptomycin at 37°C.

Prior to use in the experiments, suspension cells were resuspended in fresh medium at $5 \times 10^5$ cells/ml. As to adherent cells, fresh medium was added at a cell confluency of 70-80%.

Actinomycin D (hereinafter referred to as ActD, Sigma, St. Louis, MO, U.S.A.) was dissolved in water to a concentration of 200 $\mu$g/ml and added to media at the concentrations indicated in the examples which appear hereinafter. After incubation with or without ActD, suspension cells were collected by centrifugation, while adherent cells were harvested from both the trypsin-treated adherent cells and the culture medium.

Cell viability was evaluated by the ability to exclude trypan blue. Cell morphology was evaluated by microscopic examination of the trypan blue-treated cell culture and either the ordinary Giemsa-stained smear preparation or the smear preparation obtained by cytospin-centrifugation.

Human placental tissue was obtained from Woden Valley Hospital, Canberra, Australia.

2) cDNA probe

In addition to the <u>nbl</u> and <u>myc</u> probes described in Example 1-(3), the following probes were used.

18S ribosomal DNA probe:

A 0.97 kb <u>Pst</u>I fragment derived from plasmid pX1r14F (Maden, B. E. H., Nature, <u>288</u>, 293-296 (1980))

Ubiquitin probe:

A 1.04 kb <u>Dra</u>I-<u>Bam</u>HI fragment of human UbB gene [Baker, R. T. and Board, p. G., Nuclic Acids Res., <u>15</u>, 443 (1987)]

Furthermore, a 1.0 kb human glyceraldehyde-3-phosphate dehydrogenase cDNA was purchased from Clonetech (Palo Alto CA, U.S.A).

The human $\beta$-actin probe was a 0.4 kb <u>Eco</u>RI fragment derived from plasmid pHF$\beta$A-3' UT-HT (Ponte, P., et al., Mol. Cell. Biol., <u>3</u>, 1783-1791 (1983)).

3) Isolation and analysis of RNA and DNA

The isolation and Northern blot analysis of RNA were carried out in the same manner as described hereinbefore in (3) [Naora, H., et al., J. Immunol., 152, 5691-5702 (1994)].

PhosphorImager (Molecular Dynamics; Sunny Valley, CA, U.S.A) analysis was used to quantitate the relative intensity of hybridization signals on Northern blots.

Isolation of the chromosomal DNA and quantitation of DNA fragmentation on agarose electrophoresis gels were carried out by the techniques described hereinbefore in (2) [Naora, H., et al., Immunology, 85, 63-68 (1995)].

4) Induction of apoptosis with ActD in HL-60 cell line

HL-60 cell line was used as a model system for investigating the apoptosis induced by inhibition of gene expression.

In this model system, apoptosis is strongly induced by the transcription inhibitor ActD [Martin, S. J., et al., J. Immunol., 145, 1859-1867 (1990); Naora, H., B.B.R.C., 211, 491-496 (1995)].

In a preliminary test, the optimal conditions for the induction of apoptosis were explored by microscopic examination of trypan blue-stained cultures. After 6 hours of treatment with 1 $\mu$g/ml of ActD, approximately one-half of HL-60 cells had apparently shrunken with the formation of protuberances or were refractile in appearance in a considerable way. These cells maintain membrane integrity to a comparatively late stage, as claimed to be a feature of apoptotic cells [Wyllie, A. H. et al., Int. Rev. Cytol., 68, 251-306 (1980)], and excluded trypan blue stain .

Furthermore, processes suggesting typical apoptotic elements appeared on the cell surface just as reported previously (Tanaka, Y., et al., Exp. Cell Res., 213, 242-252 (1994)).

The assessment procedure for apoptotic morphologies was supported by the evidence obtained in several systems as mentioned above. As reported by Matsubara and coworkers (Matsubara, K., et al., Exp. Cell Res., 213, 412-417 (1994)), chromatin condensation either around the nuclear membrane or in the intracellular mass was observed in the Giemsa-stained cell preparations. The time course of apoptotic morphology highly correlated with the time course of internucleosomal DNA fragmentation. Induction of internucleosomal DNA fragmentation was maximal after 6 hours of treatment with 1 $\mu$g/ml of ActD when cells were incubated for 3, 6, 9, 12 and 18 hours. Prolongation of treatment time beyond 6 hours resulted in a diminution of the "DNA ladder" pattern and a decrease in cell viability. Incubation at the ActD concentration of 0.5-10 $\mu$g/ml modestly increased the proportion of cells with apoptotic morphology and the degree of "DNA ladder" formation observed at 6 hours.

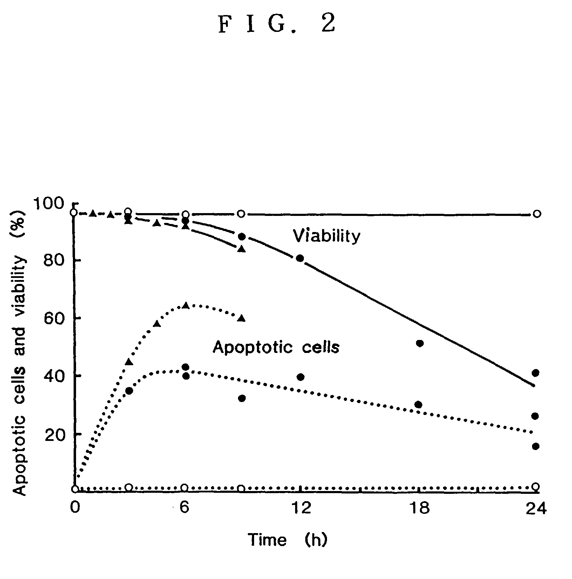

These results are diagrammatically illustrated in Fig. 2.

Fig. 2 is a graph showing the results obtained by investigating ActD-induced apoptosis using HL-60 cell line, in which the open circle represents untreated HL-60 cells, the closed circle represents HL-60 cells incubated in the presence of ActD (1 $\mu$g/ml), and the closed triangle represents those cells which were incubated in the presence of ActD (5 $\mu$g/ml). This graph shows the proportion of apoptotic cells and the cell viability (%) (ordinate) as plotted against incubation time (abscissa) up to 24 hours. The viability (survival rate) of cells was assessed by the ability to exclude trypan blue stain and the proportion of apoptotic cells was estimated from the ratio of cells showing surface protuberances, which are a morphological feature of apoptotic cells, cellular shrinkage, and high refractile properties to cells not exhibiting such characteristics.

As also shown in Fig. 2, increase of ActD concentration within the range of 1-5 $\mu$g/ml did not affect the time course of induction of apoptotic morphology to any remarkable extent. No change was found in the time course of "DNA ladder" formation, either.

Therefore, as the assay conditions, 6-hour incubation at the ActD concentration of 1 $\mu$g/ml was employed. The above condition was used in the subsequent experiments unless otherwise noted.

5) Induction of apoptosis by ActD in other cell lines

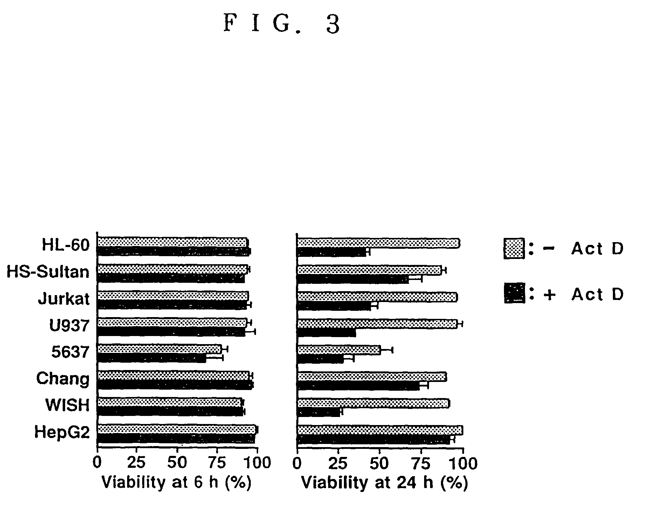

The ability of ActD to induce apoptosis in other human cell lines was investigated. The viability (the data presented in Fig. 3) of cells in cultures was evaluated after 6-hour and 24-hour incubation with (+) and without (-) ActD (1 $\mu$g/ml). Moreover, the DNA and RNA isolated from each cell line incubated with and without 1 $\mu$g/ml or 10 $\mu$g/ml of ActD for 0, 6 and 24 hours were respectively analyzed.

The results obtained by investigating the viability of cells using, as human cell lines, HL-60, HS-Sultan, Jurkat, U937, 5637, Chang, WISH and HepG2 are shown in Fig. 3. In the graph, the viability of cells in cultures after 6 hours of incubation are shown in the left column and the viability after 24 hours of incubation are shown in the right column. The solid and dotted bars represent the results obtained in the presence and the absence of ActD, respectively.

The 6-hours ActD treatment of T-lymphoblastic leukemia cell line Jurkat and histiocytic leukemia cell line U937 induced substantial internucleosomal DNA fragmentation in these cells. However, the degree of induction was less than that in HL-60 cells. Moreover, no significant effect was found on the viability of cells.

In HS-Sultan, a lesser, though considarable degree of the "DNA ladder" formation was also induced under the same conditions. Formation of the "DNA ladder" in these cells correlated with cellular shrinkage, refractile features, and exclusion of trypan blue. The prolonged treatment for 24 hours reduced the viability of these cells.

The susceptibility of cells to apoptotic induction by ActD was not restricted to cells derived from the hematopoietic system and to suspension cell cultures. In experiments using human adherent cells, the DNA from the cells pooled from the trypsin-treated monolayer and the culture medium was analyzed.

The morphology of apoptosis was investigated by criteria different from that used for suspension cells.

Other studies have been described an early phase of apoptosis in adherent cell types to involve gradual rounding and detaching of cells from each other, but not from the flask wall and the appearance of large DNA fragments of 23 to 50 kb were reported [Desjardins, L. M. and MacManus, J. P., Exp. Cell Res., 216, 380-387 (1984)].

With the progress of apoptosis, cells began to shrink and become detached from the flask and contained the "DNA ladder" [Desjardins, L. M. and MacManus, J. P., Exp. Cell Res., 216, 380-387 (1984): Kanter, P., et al., B.B.R.C., 118, 392-399 (1984)].

Bladder cancer cell 5637 line showed an considerable amount of internucleosomal DNA fragmentation after 6 hours of incubation in the presence of ActD.

This finding was correlated with the appearance of floating cells. The 5637 cells which remained attached to the flask wall, displayed the "rounded" appearance and were separated from one another.

Chang liver cells contained the "DNA ladder" to a smaller extent.

In contrast, even when treated with, for example, ActD at a 10-fold higher concentration for 6 hours, neither WISH amnionic cells nor liver cancer HepG2 cells showed internucleosomal DNA fragmentation. For both lines of cells, little change in morphology was found from their untreated control.

After 24 hours of ActD treatment, WISH cells showed a substantial loss of viability (Fig. 3) and almost all the cells were floating, as was seen in the culture of 5637 cells and Chang cells.

However, WISH cells showed the "smear"-like DNA pattern which is distinct from the "DNA ladder". This pattern suggested that these cells might undergo a process of cell death different from the process of death which has been observed with 5637 and Chang cells and several other adherent cell lines [Desjardins, L. M. and MacManus, J. P., Exp. Cell Res., 216, 380-387 (1995): Kanter, P., et al., Biochem. Biophys. Res. Comm., 118, 392-399 (1984)].

Similarly, the majority of HepG2 cells were freely floating at 24 hours after ActD treatment, and showed the "smear" DNA pattern instead of "the DNA ladder". However, the morphology of floating HepG2 cells was more "rounded" and these cells were more viable than WISH cells (Fig. 3).

6) Constitutive nbl expression level and induction of apoptosis

Constitutive nbl expression levels in various human cell lines were determined by PhosphorImager analysis.

As demonstrated in Example 1-(4), normal mouse tissues such as the cerebellum, liver and lung possess nbl mRNA at low levels.

In this experiment, the level of nbl mRNA in the normal human placenta was taken as a standard for comparing the relative nbl mRNA levels in a variety of cell lines.

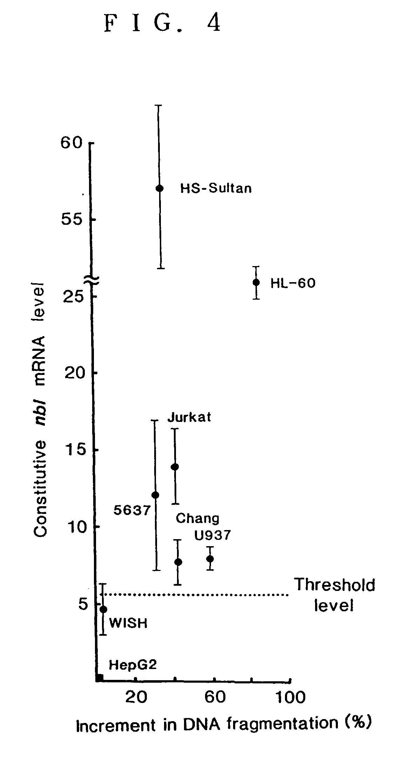

The results are shown in Fig. 4. In the diagram, the extent of DNA fragmentation (%) on the abscissa is plotted against the level of constitutive nbl mRNA expression on the ordinate for each cell line.

The nbl mRNA level in each cell line represents the amount of expression relative to the nbl mRNA level in human placenta and this value was corrected for RNA content. For example, the constitutive nbl mRNA level in a given cell line (C) is calculated from $(Nc/Np) \times (Rp/Rc)$. Nc and Np represent the intensities of nbl hybridization signals in test cell line C and placenta, respectively, and Rc and Rp represent the intensities of 18Sr RNA hybridization signals in test cell line C and placenta, respectively.

The degree of DNA fragmentation, induced at 6 hours after treatment with ActD (1 μg/ml) was quantified as described in the methods and expressed as an increment above the low basal level of DNA fragmentation detected in untreated cells.

The constitutive nbl mRNA level was expressed as the mean±SD of 3-4 replicate experiments and the value was plotted against the increment in DNA fragmentation.

It was shown that ActD-induced apoptosis had occurred when nbl was constitutively expressed above the threshold level indicated by the dotted line.

It is apparent from the above diagram that the constitutive nbl mRNA levels detected in untreated HL-60 and HS-Sultan cell lines were as high as 26- and 57-fold, respectively, the level detected in human placenta.

The constitutive level of nbl mRNA was also relatively high in Chang, U937, 5637, and Jurkat cell lines, ranging from about 8- to about 14-fold the placental level.

The nbl mRNA level was very low in WISH cell line and the level in HepG2 cell line was lower than the placental level.

Generally speaking, cells with higher levels of constitutive nbl expression tended to show the typical apoptotic morphology and internucleosomal DNA fragmentation to a greater extent in response to ActD.

It can also be seen in Fig. 4 that cells with relatively low constitutive nbl expression levels, such as WISH and HepG2 cell lines, did not show typical apoptotic morphology or "DNA ladder" formation to any significant extent but exhibited the "smear" DNA pattern only after prolonged ActD treatment.

The correlation between the level of constitutive nbl expression and the induction of apoptosis is not strictly proportional but susceptibility of cells to ActD-induced apoptosis appears to initially involve high constitutive levels of nbl expression above a certain threshold. The threshold mRNA level seems to be about 5- to 6-fold the human placental nbl mRNA level.

7) Induction of apoptosis and suppression of nbl expression

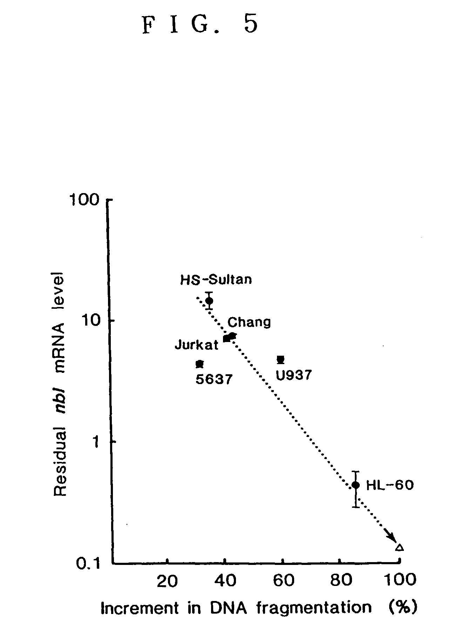

In this experiment, the residual nbl mRNA level in cells at 6 hours after addition of ActD (1 µg/ml) was assayed by PhosphorImager analysis and the value was expressed in relation to the nbl mRNA level in the human placenta. The results are shown in Fig. 5.

It should be noted that, as in the case of Fig. 4, the above-mentioned residual value was corrected for RNA content and, then, plotted against the increment in induced DNA fragmentation at 6 hours after addition of ActD. The residual nbl mRNA level was expressed as the mean±SD of 3-4 replicate experiments. In the diagram, the dotted line represents the statistically calculated regression curve and △ represents the value of the nbl mRNA level obtained by extrapolation (0.12) when all the DNA was fragmented in apoptotic cells.

It can be seen from Fig. 5 that in the HL-60 cells at 6 hours after ActD treatment, almost no nbl transcript could be detected. Though to a lesser degree than in HL-60 cell line, decreases in nbl expression in response to ActD were also observed in HS-Sultan, Chang, U937, and Jurkat cell lines. The change in nbl mRNA level was almost negligible in WISH cells.

On the other hand, no significant decrease in nbl expression was found in HepG2 cells even at a high concentration of ActD. Moreover, in WISH cell and HepG2 cell lines, decreases in the nbl expression were found only after prolonged (24 hr-long) ActD treatment. However, this decrease coincided with a decline in levels of stable mRNA such as β-actin [Naora, H., B.B.R.C., 211, 491-496 (1995)] and glyceraldehyde-3-phosphate dehydrogenase mRNA.

Generally speaking, cells with high constitutive nbl expression levels not only showed the typical apoptotic morphology and "DNA ladder" formation to a greater extent in the response to ActD, but also tended to reveal a greater inhibition of nbl expression by ActD.

However, as a notable exception to this relationship, HS-Sultan cell line with a very high level of constitutive nbl expression showed a relatively large decline in nbl mRNA level following addition of ActD. However, when compared with the amounts of "DNA ladder" formation observed in Jurkat, U937 and other cell lines showing constitutive nbl expression levels and the degree of nbl inhibition lower than the constitutive nbl expression level and degree of nbl inhibition observed in the HS-Sultan cell line, the degree of ActD-induced internucleosomal DNA fragmentation in the HS-Sultan cell line was not high.

When compared with the residual nbl mRNA levels in HL-60, Jurkat, U937, and other cell lines which showed greater amounts of "DNA ladder" formation, the residual nbl mRNA level detected in the HS-Sultan cell line at 6 hours after ActD treatment was still substantially high (Fig. 5). The ActD-induced decline of the nbl expression level from the high initial constitutive level of nbl expression is considered to be an important factor in the phenomenon of internucleosomal DNA fragmentation.

The above results indicate the relation between a suppression of the high constitutive nbl expression and the induction of ActD-induced apoptosis. The differential effects of ActD on various cell lines are not due to possible variations in the ability of ActD to penetrate effectively or inhibit transcription in different cell types.

Firstly, apoptosis and the change in nbl expression were induced in varying degrees in not only suspension cells but also adherent cells.

Secondly, the decline in myc mRNA level observed after ActD treatment did not vary significantly between cell lines which showed substantial differences in the change in nbl expression and in the degree of DNA fragmentation.

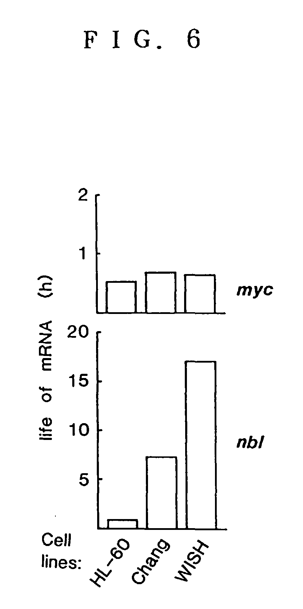

Fig. 6 is a graph showing the half-lives of the nbl and myc mRNAs in HL-60, Chang, and WISH cell lines estimated from the regression curves calculated by PhosphorImager analysis of the nbl and myc mRNA hybridization signals on the Northern blots of RNA. These RNA were isolated from the cells incubated with ActD (5 µg/ml) for 0, 1, 2, 3, 4.5 and 6 hours.

The measured half-lives of myc mRNA in these cells were consistent with the values reported in other types of cells [Rabbitts, P. H., et al., EMBO J., 4, 3727-3733 (1985)].

In the case of HepG2 cell line manifesting only the least induction of apoptosis upon ActD treatment, the extremely low myc mRNA level made it difficult to measure the ActD-induced suppression of the myc mRNA level. Therefore, it was tested whether the expression of genes other than myc would be inhibited by ActD in this cell line.

The ActD-induced suppression of the primary ubiquitin transcript level was found in HepG2 cell line to be almost the same extent as in HL-60 cell line, indicating that ActD is as effective in HepG2 cell line, too.

8) Specific effects on apoptotic induction by nbl antisense oligonucleotides

In order to analyze whether there is any specific causal relationship between inhibition of high nbl expression and induction of apoptosis and whether inhibition of nbl expression merely coincides with induction of apoptosis or is a consequence of induced apoptosis, HL-60 cells were incubated with nbl antisense oligomers within the concentration and time range commonly used in other studies [Shi, Y., et al., Science, 257, 212-214 (1992); Kimura, S., et al., Cancer Res., 55, 1379-1384 (1995)].

The phosphorothioate oligonucleotides synthesized at Biomolecular Resource Facility (The National University of Australia, Canberra) and purified by reverse-phase HPLC were used.

The sequence of the nbl antisense oligomer is complementary to the ATG initiation codon and the downstream 4 codons of nbl mRNA as shown in SEQ ID NO:4.

As control, an oligomer of SEQ ID NO:5 comprising a random sequence of the same nucleotide composition as the nbl antisense oligomer was used.

In addition, an nbl sense oligomer containing the sequence of the initiation codon and downstream 4 codons of nbl mRNA was also used in the experiment.

Oligomers were dissolved in water and added, at the final concentration of 20 $\mu$M, to the cells resuspended in fresh medium at $1 \times 10^5$ cells/ml in a 96-well microtiter plate and the plate was incubated for 2 days.

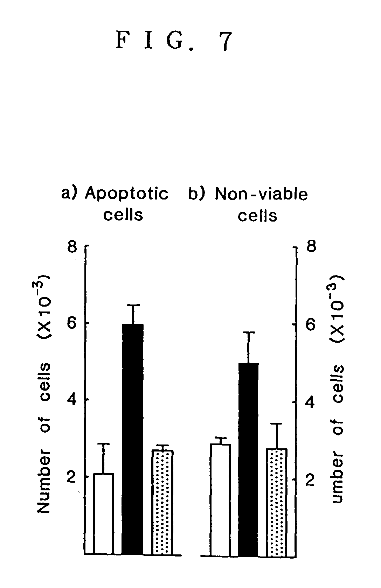

The results are shown in Fig. 7.

Fig. 7 is a graph showing the effects of the nbl antisense oligomers on the apoptosis and viability of HL-60 cells, where the open bar represents HL-60 cells in the absence of any oligomers, the solid bar represents the same cells in the presence of 20 $\mu$M nbl antisense oligomers, and the dotted bar represents the same cells in the presence of 20 $\mu$M random oligomers.

The number (a) of apoptotic cells showing protuberances, shrinkage and which were refractile in appearance and the number (b) of non-viable cells were counted. The results of two experiments were expressed in means±SD.

The HL-60 cells incubated in the presence of the nbl antisense oligomers were shrivelled and refractile with many demonstrating protuberances characteristic of apoptotic bodies as described earlier. A corresponding increase in non-viable cells was also observed.

Moreover, the appearance of apoptotic cells and non-viable cells was induced in a time- and nbl antisense oligomer concentration-dependent manner.

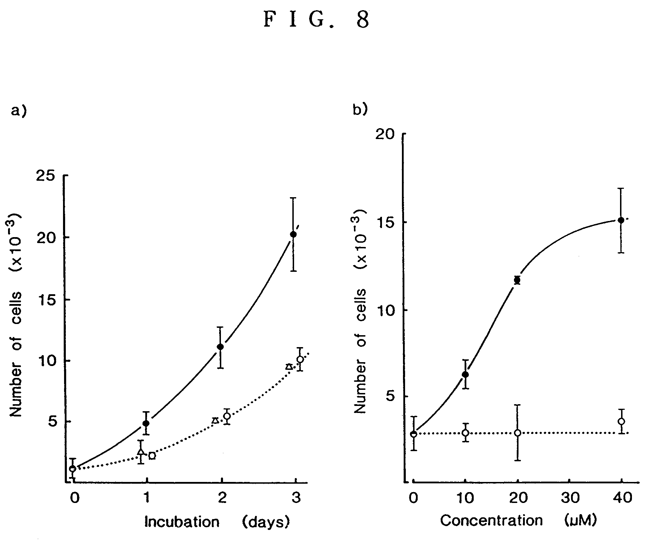

The above findings are shown in Fig. 8.

Fig. 8-a) shows the total numbers of non-viable cells plus apoptotic cells which appeared when HL-60 cells were incubated in the absence of any oligomers ($\triangle$), in the presence of 20 $\mu$M nbl antisense oligomers (solid circle), or in the presence of 20 $\mu$M random oligomers (open circle) for 1, 2 and 3 days under the same conditions as in the case of Fig. 6.

Fig. 8-b) shows the total numbers of apoptotic and non-viable HL-60 cells after 2 days of incubation in the presence of the nbl antisense oligomers (solid circle) or the random oligomers (open circle) at the concentrations indicated, respectively.

It is apparent from Fig. 8-a) and -b) that the appearance of apoptotic cells and non-viable cells was induced by the nbl antisense oligomers in a concentration and time-dependent manner.

In contrast, HL-60 cells incubated in the presence of phosphorothioate oligomers having the random sequence of the same nucleotide composition as that of the nbl antisense oligomers did not show any significant difference from HL-60 cells incubated in the absence of any oligomers in terms of morphology and viability.

Incubation in the presence of the nbl sense oligomers containing the ATG initiation codon and downstream 4 codons of nbl mRNA gave results similar to those observed with the random-sequence oligomers.

It was further investigated whether DNA fragmentation could be induced in HL-60 cells when the cells were incubated in the presence of the nbl antisense oligomers. For this investigation, in consideration of the small cell population for oligomer experiments, DNA cleavage was detected by labeling the free 3'-OH ends of DNA with terminal deoxynucleotidyl transferase in accordance with the method of Kimura and coworkers [Kimura, S., et al., Cancer Res., 55, 1379-1384 (1995)].

The cells with labeled DNA were precipitated and collected on a nitrocellulose filter using cold 10% trichloroacetic acid. The degree of DNA fragmentation was assessed by scintillation counting. HL-60 cells were incubated in the absence of any oligomers or in the presence of 20 $\mu$M nbl antisense oligomers or 20 $\mu$M random oligomer for 2 days. The cells were then fixed in formaldehyde by method of Gorczyca et al. [Gorczyca, W., et al., Cancer Res., 53, 1945-1951 (1993)].

The free 3'-OH ends on isolated chromosomal DNA were also labeled using the 3'-end DNA labeling kit (Boehringer-Mannheim, Germany). The labeled DNA was electrophoresed on 6% polyacrylamide/8M urea gel and the degree of fragmentation was determined by PhosphorImager analysis of the radiolabeled DNA pattern on the dry gel.

The result showed no significant difference between the amount of DNA fragmentation detected in the HL-60 cells incubated in the absence of oligomers and that of the cells incubated in the presence of the random-sequence oligomers. In contrast, in the presence of the nbl antisense oligomers, the degree of DNA fragmentation in HL-60 cells after 2 days of incubation showed a 4-fold increase. The DNA fragmentation observed by labeling fragments in the isolated chromosomal DNA was similar to the result obtained by in situ labeling in fixed cells. Therefore, it is suggested that the nbl antisense oligomers specifically induced the appearance of apoptotic morphology and DNA fragmentation and led to loss of viability in HL-60 cells.

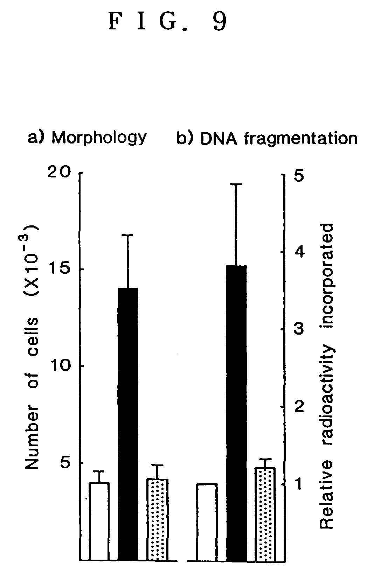

The above conclusion is seen in Fig. 9 showing the DNA fragmentation induced by the nbl antisense oligomers. Fig. 9-a is a graph showing the combined total number of apoptotic cells and non-viable cells after 2 days of incubation of HL-60 cells in the absence of any oligomers (open bar) or in the presence of 20 $\mu$M nbl antisense oligom-

ers (solid bar) or 20 μM random oligomers (dotted bar). The degree of fragmentation in the isolated chromosomal DNA radiolabeled with terminal deoxynucleotidyl transferase is shown in Fig. 9-b. The results are expressed in mean±SD.

INDUSTRIAL APPLICABILITY

The apoptosis-associated gene of the present invention is capable of inducing or inhibiting induction of apoptosis through appropriate regulation of its expression and can, therefore, be used effectively for cancer therapy, among other applications.

**Claims**

1. An apoptosis-associated gene which comprises a nucleotide sequence coding for the amino acid sequence of SEQ ID NO:1.

2. A method of inducing apoptosis in a cell comprising a step of stimulating the cell to express an apoptosis-associated gene and a subsequent step of inhibiting expression of the said apoptosis-associated gene.

3. A method according to Claim 2 comprising elevating apoptosis-associated gene expression to a level not lower than an induction threshold value in the apoptosis-associated gene expression step and inhibiting the expression to a level not higher than a threshold value in the apoptosis-associated gene expression inhibiting step.

4. A method according to Claim 3 wherein the said induction threshold value is approximately 5 times the apoptosis-associated gene mRNA expression level value in normal tissues.

5. A method according to Claim 3 or 4 wherein the said apoptosis-associated gene is selected from among nbl gene, MFT-1 gene, fte-1 gene, RPS3a gene, TU-11 gene, KRP-A gene and cyc07 gene.

6. An apoptosis-inducing agent comprising an apoptosis-associated gene-incorporated retrovirus as an active ingredient.

7. An apoptosis inducing agent according to Claim 6 wherein the said apoptosis-associated gene is selected from among nbl gene, MFT-1 gene, fte-1 gene, RPS3a gene, TU-11 gene, KRP-A gene and cyc07 gene.

8. An apoptosis inducing agent according to Claim 6 wherein the said apoptosis-associated gene is nbl gene.

9. An apoptosis-inducing pharmaceutical composition for inhibiting a high apoptosis-associated gene expression level in a cell to an apoptosis induction level to thereby induce apoptosis, which comprises an apoptosis-associated gene antisense as an active ingredient.

10. A therapeutic agent for herpesvirus infection comprising using the apoptosis inducing agent claimed in Claim 6 in the treatment of herpesvirus infection.

11. Use of a specific gene and an antisense to the said gene characterized by elevating expression of an apoptosis-associated gene to the above induvtion threshold value and, then, suppressing expression of the said gene to a level below a threshold value to induce apoptosis.

12. A method of arresting apoptosis comprising maintaining a cell showing a high apoptosis-associated gene expression level so that the expression level will not fall below a threshold value to thereby arrest induction of apoptosis.

13. An apoptosis arresting agent for arresting apoptosis by maintaining a cell showing a high apoptosis-associated gene expression level so that the expression level will not fall below a threshold level.

14. An apoptosis arresting agent according to Claim 13 comprising an apoptosis-associated gene-incorporated retrovirus as an active ingredient.

15. The arresting agent of Claim 13 wherein the said apoptosis-associated gene is at least one member selected from among nbl gene, MFT-1 gene, fte-1 gene, RPS3a gene TU-11 gene, KRP-A gene and cyc07 gene.

16. A method for treating HIV infection (asymptomatic stage) and neuropathy associated with nerve cell death by utilizing the method claimed in Claim 12.

17. A method for treating HIV infection (asymptomatic stage) and neuropathy associated with nerve cell death by utilizing the arresting agent claimed in Claim 13.

18. An apoptosis arresting agent according to Claim 13 comprising the nbl-associated gene-incorporated retrovirus as an active ingredient.

19. A method of measuring an apoptosis-associated gene expression level using an apoptosis induction or inhibition marker.

20. An apoptosis induction or inhibition marker comprising as an active ingredient at least one member selected from among fragments of at least one gene selected from among nbl gene, MFT-1 gene, fte-1 gene, RPS3a gene, TU-11 gene, KRP-A gene and cyc07 gene, antibodies to proteins produced by expression of the said genes, and fragments of said antibodies.

21. An apoptosis-associated gene expression arresting agent for arresting expression of an apoptosis-associated gene.

22. An arresting method according to Claim 12 for arresting expression of at least one gene selected from among nbl gene, MFT-1 gene, fte-1 gene, RPS3a gene, TU-11 gene, KRP-A gene and cyc07 gene.

23. An arresting method according to Claim 12 for inhibiting translation from the mRNA of at least one gene selected from among nbl gene, MFT-1 gene, fte-1 gene, RPS3a gene, TU-11 gene, KRP-A gene and cyc07 gene.

24. An arresting method according to Claim 12 for arresting transcription from the DNA to RNA of at least one gene selected from among nbl gene, MFT-1 gene, fte-1 gene, RPS3a gene TU-11 gene, KRP-A gene and cyc07 gene.

25. A method for cancer therapy comprising inhibiting a high apoptosis-associated gene expression in a cell to the apoptosis induction level to induce apoptosis and thereby cure cancer.

26. An anticancer agent for use in the method claimed in Claim 25.

27. A method for cancer therapy according to Claim 25 comprising using an apoptosis-associated gene antisense.

28. The anticancer agent of Claim 26 which is an apoptosis-associated gene antisense.

29. A method for preventing induction of apoptosis which comprises suppressing and/or maintaining the amount of expression of an apoptosis-associated gene in a cell so that it will not increase over the threshold level.

30. An apoptosis induction preventing agent for suppressing and/or maintaining the amount of an apoptosis-associated gene in a cell so that it will not increase over the threshold level.

31. A detection method for detecting an apoptosis-associated gene product released from a cell showing an amount of expression of an intracellular apoptosis- associated gene over the threshold value and/or a cell expressing an apoptosis-associated gene with the aid of an antibody thereto and a detection method for detecting the mRNA level of the said gene using such a gene as a probe.

32. A detection method according to Claim 31 for detecting human cancer cells and/or lymphocytes in a patient with HIV infection.

FIG. 1

Hours after administration

of Dexamethasone

F I G. 2

# F I G. 3

F I G. 4

# F I G. 5

Increment in DNA fragmentation (%)

F I G. 6

F I G. 7

a) Apoptotic b) Non-viable
   cells       cells

F I G. 8

FIG. 9

a) Morphology     b) DNA fragmentation

## INTERNATIONAL SEARCH REPORT

| International application No. |
| --- |
| PCT/JP95/02090 |

**A. CLASSIFICATION OF SUBJECT MATTER**

Int. Cl$^6$ C12N15/12, C12N7/01, A61K35/76, A61K48/00, C12Q1/68, G01N33/563, C07K16/18

According to International Patent Classification (IPC) or to both national classification and IPC

**B. FIELDS SEARCHED**

Minimum documentation searched (classification system followed by classification symbols)

Int. Cl$^6$ C12N15/12, C12N7/01, A61K35/76, A61K48/00, C12Q1/68, G01N33/563, C07K16/18

Documentation searched other than minimum documentation to the extent that such documents are included in the fields searched

Electronic data base consulted during the international search (name of data base and, where practicable, search terms used)

CAS ONLINE, WPI, WPI/L, BIOSIS PREVIEWS

**C. DOCUMENTS CONSIDERED TO BE RELEVANT**

| Category* | Citation of document, with indication, where appropriate, of the relevant passages | Relevant to claim No. |
| --- | --- | --- |
| P,X | Naora, H. et al. "Association of nbl gene expression and glucocorticoid-induced apoptosis in mouse thymus in vivo" Immunology (1995 May) Vol. 85, No. 1, p. 63-68 | 1-15, 18-24, 26, 28-32 |
| P,X | WO, 95/00642, A1 (Arch Dev. Corp.), January 5, 1995 (05. 01. 95) & AU, 9471773, A | 2-4, 6, 9-14, 19, 21, 26, 28, 29-32 |
| P,X | WO, 95/15084, A1 (LXR Biotechnology Inc.), June 8, 1995 (08. 06. 95) & AU, 9513351, A | 2-4, 6, 9-14, 19, 21, 26, 28, 29-32 |
| P,X | WO, 95/13292, A1 (La Jolla Cancer Res. Found.), May 18, 1995 (18. 05. 95) & AU, 9511742, A | 2-4, 6, 9-14, 19, 21, 26, 28, 29-32 |
| X | WO, 93/24653, A1 (Consiglio Naz Delle Richerche), December 9, 1993 (09. 12. 93) | 2-4, 9, 11, 21, 26, 28 |

| ☒ Further documents are listed in the continuation of Box C. | ☐ See patent family annex. |
| --- | --- |

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| January 30, 1996 (30. 01. 96) | February 20, 1996 (20. 02. 96) |

| Name and mailing address of the ISA/ | Authorized officer |
| --- | --- |
| Japanese Patent Office | |
| Facsimile No. | Telephone No. |

Form PCT/ISA/210 (second sheet) (July 1992)

31

**EP 0 781 844 A1**

## INTERNATIONAL SEARCH REPORT

| International application No. |
|---|
| PCT/JP95/02090 |

C (Continuation).   DOCUMENTS CONSIDERED TO BE RELEVANT

| Category* | Citation of document, with indication, where appropriate, of the relevant passages | Relevant to claim No. |
|---|---|---|
| X | WO, 93/20200, A1 (Imperial Cancer Res. Technology), October 14, 1993 (14. 10. 93) & EP, 633934, A1 | 2-4, 9, 11, 21, 26, 28 |
| P,X | Tatsuya Ito et al. "bcl-2 Gene and Apoptosis" Weekly Igaku no Ayumi (June 3, 1995) Vol. 173, No. 10, p. 803-807 | 2-4, 6, 9-14, 19, 21, 26, 28, 29-32 |
| X | Tatsuya Ito et al. "bcl-2 Gene and Apoptosis" Weekly Igaku no Ayumi (May 14, 1994) Vol 169, No. 7, p. 785-789 | 2-4, 6, 9-14, 19, 21, 26, 28, 29-32 |

Form PCT/ISA/210 (continuation of second sheet) (July 1992)

32

**INTERNATIONAL SEARCH REPORT**

| International application No. |
| --- |
| PCT/JP95/02090 |

---

**Box I**    Observations where certain claims were found unsearchable (Continuation of Item 1 of first sheet)

This international search report has not been established in respect of certain claims under Article 17(2)(a) for the following reasons:

1. [X]   Claims Nos.:   16, 17, 25, 27
   because they relate to subject matter not required to be searched by this Authority, namely:

   Claims 16, 17, 25 and 27 are considered to be related to

   methods for treatment of the human body by therapy.

2. [ ]   Claims Nos.:
   because they relate to parts of the international application that do not comply with the prescribed requirements to such an extent that no meaningful international search can be carried out, specifically:

3. [ ]   Claims Nos.:
   because they are dependent claims and are not drafted in accordance with the second and third sentences of Rule 6.4(a).

---

**Box II**    Observations where unity of invention is lacking (Continuation of Item 2 of first sheet)

This International Searching Authority found multiple inventions in this international application, as follows:

1. [ ]   As all required additional search fees were timely paid by the applicant, this international search report covers all searchable claims.

2. [ ]   As all searchable claims could be searched without effort justifying an additional fee, this Authority did not invite payment of any additional fee.

3. [ ]   As only some of the required additional search fees were timely paid by the applicant, this international search report covers only those claims for which fees were paid, specifically claims Nos.:

4. [ ]   No required additional search fees were timely paid by the applicant. Consequently, this international search report is restricted to the invention first mentioned in the claims; it is covered by claims Nos.:

**Remark on Protest**    [ ]   The additional search fees were accompanied by the applicant's protest.

                                 [ ]   No protest accompanied the payment of additional search fees.

Form PCT/ISA/210 (continuation of first sheet (1)) (July 1992)

SEQUENCE LISTING

(2) INFORMATION FOR SEQ ID NO:1:

    (i) SEQUENCE CHARACTERISTICS:
        (A) LENGTH: 264 amino acids
        (B) TYPE: amino acid
        (D) TOPOLOGY: linear

    (ii) MOLECULE TYPE: protein

    (xi) SEQUENCE DESCRIPTION: SEQ ID NO:1:

```
Met Ala Val Gly Lys Asn Lys Arg Leu Thr Lys Gly Gly Lys Lys Gly
 1               5                  10                  15

Ala Lys Lys Lys Val Val Asp Pro Phe Ser Lys Lys Asp Trp Tyr Asp
            20                  25                  30

Val Lys Ala Pro Ala Met Phe Asn Ile Arg Asn Ile Gly Lys Thr Leu
        35                  40                  45

Val Thr Arg Thr Gln Gly Thr Lys Ile Ala Ser Asp Gly Leu Lys Gly
     50                  55                  60

Arg Val Phe Glu Val Ser Leu Ala Asp Leu Gln Asn Asp Glu Val Ala
  65                  70                  75                  80

Phe Arg Lys Phe Lys Leu Ile Thr Glu Asp Val Gln Gly Lys Asn Cys
             85                  90                  95

Leu Thr Asn Phe His Gly Met Asp Leu Thr Arg Asp Lys Met Cys Ser
             100                 105                 110

Met Val Lys Lys Trp Gln Thr Met Ile Glu Ala His Val Asp Val Lys
         115                 120                 125

Thr Thr Asp Gly Tyr Leu Leu Arg Leu Phe Cys Val Gly Phe Thr Lys
     130                 135                 140

Lys Arg Asn Asn Gln Ile Arg Lys Thr Ser Tyr Ala Gln His Gln Gln
145                 150                 155                 160

Val Arg Gln Ile Arg Lys Lys Met Met Glu Ile Met Thr Arg Glu Val
             165                 170                 175
```

```
Gln Thr Asn Asp Leu Lys Glu Val Val Asn Lys Leu Ile Pro Asp Ser
            180                 185                 190

Ile Gly Lys Gly Ile Glu Lys Ala Cys Gln Ser Ile Tyr Pro Leu His
        195                 200                 205

Asp Val Phe Val Arg Lys Val Lys Met Leu Lys Lys Pro Lys Phe Glu
    210                 215                 220

Leu Gly Lys Leu Met Glu Leu His Gly Glu Gly Ser Ser Ser Gly Lys
225                 230                 235                 240

Ala Thr Gly Asp Glu Thr Gly Ala Lys Val Glu Arg Ala Asp Gly Tyr
                245                 250                 255

Glu Pro Pro Val Gln Glu Ser Val
            260
```

(2) INFORMATION FOR SEQ ID NO:2:

    (i) SEQUENCE CHARACTERISTICS:
        (A) LENGTH: 792 base pairs
        (B) TYPE: nucleic acid
        (C) STRANDEDNESS: double
        (D) TOPOLOGY: linear

    (ii) MOLECULE TYPE: CDS


    (xi) SEQUENCE DESCRIPTION: SEQ ID NO:2:

```
ATGGCGGTTG GCAAGAACAA GCGCCTTACG AAAGGCGGCA AAAAGGGAGC CAAGAAGAAA    60

GTGGTTGATC CATTTTCTAA GAAAGATTGG TATGATGTGA AAGCACCTGC TATGTTCAAT   120

ATAAGAAATA TTGGAAAGAC GCTCGTCACC AGGACCCAAG GAACCAAAAT TGCATCTGAT   180

GGTCTCAAGG GTCGTGTGTT TGAAGTGAGT CTTGCTGATT TGCAGAATGA TGAAGTTGCA   240

TTTAGAAAAT TCAAGCTGAT TACTGAAGAT GTTCAGGGTA AAAACTGCCT GACTAACTTC   300

CATGGCATGG ATCTTACCCG TGACAAAATG TGTTCCATGG TCAAAAAATG GCAGACAATG   360

ATTGAAGCTC ACGTTGATGT CAAGACTACC GATGGTTACT TGCTTCGTCT GTTCTGTGTT   420

GGTTTTACTA AAAAACGCAA CAATCAGATA CGGAAGACCT CTTATGCTCA GCACCAACAG   480

GTCCGCCAAA TCCGGAAGAA GATGATGGAA ATCATGACCC GAGAGGTGCA GACAAATGAC   540
```

TTGAAAGAAG TGGTCAATAA ATTGATTCCA GACAGCATTG GAAAAGGCAT AGAAAAGGCT     600

TGCCAATCTA TTTATCCTCT CCATGATGTC TTCGTTAGAA AAGTAAAAAT GCTGAAGAAG     660

CCCAAGTTTG AATTGGGAAA GCTCATGGAG CTTCATGGTG AAGGCAGTAG TTCTGGAAAA     720

GCCACTGGGG ACGAGACAGG TGCTAAAGTT GAACGAGCTG ATGGATATGA ACCACCAGTC     780

CAAGAATCTG TT                                                         792

(2) INFORMATION FOR SEQ ID NO:3:

     (i) SEQUENCE CHARACTERISTICS:
        (A) LENGTH: 898 base pairs
        (B) TYPE: nucleic acid
        (C) STRANDEDNESS: single
        (D) TOPOLOGY: linear

    (ii) MOLECULE TYPE: cDNA

   (iii) HYPOTHETICAL: NO

    (iv) ANTI-SENSE: NO

   (vii) IMMEDIATE SOURCE:
        (A) LIBRARY: Human cDNA
        (B) CLONE: nbl

    (ix) FEATURE:
        (A) NAME/KEY: CDS
        (B) LOCATION: 34..825

    (xi) SEQUENCE DESCRIPTION: SEQ ID NO:3:

TTTTTTTTTT TTTTTGGCTC TCTGACCAGC ACC ATG GCG GTT GGC AAG AAC AAG     54
                                     Met Ala Val Gly Lys Asn Lys
                                     1             5

CGC CTT ACG AAA GGC GGC AAA AAG GGA GCC AAG AAG AAA GTG GTT GAT     102
Arg Leu Thr Lys Gly Gly Lys Lys Gly Ala Lys Lys Lys Val Val Asp
        10                  15                 20

CCA TTT TCT AAG AAA GAT TGG TAT GAT GTG AAA GCA CCT GCT ATG TTC     150
Pro Phe Ser Lys Lys Asp Trp Tyr Asp Val Lys Ala Pro Ala Met Phe
        25                  30                 35

AAT ATA AGA AAT ATT GGA AAG ACG CTC GTC ACC AGG ACC CAA GGA ACC     198
Asn Ile Arg Asn Ile Gly Lys Thr Leu Val Thr Arg Thr Gln Gly Thr

```
          40                        45                        50                        55

AAA ATT GCA TCT GAT GGT CTC AAG GGT CGT GTG TTT GAA GTG AGT CTT          246
Lys Ile Ala Ser Asp Gly Leu Lys Gly Arg Val Phe Glu Val Ser Leu
                    60                        65                        70

GCT GAT TTG CAG AAT GAT GAA GTT GCA TTT AGA AAA TTC AAG CTG ATT          294
Ala Asp Leu Gln Asn Asp Glu Val Ala Phe Arg Lys Phe Lys Leu Ile
                    75                        80                        85

ACT GAA GAT GTT CAG GGT AAA AAC TGC CTG ACT AAC TTC CAT GGC ATG          342
Thr Glu Asp Val Gln Gly Lys Asn Cys Leu Thr Asn Phe His Gly Met
                90                        95                        100

GAT CTT ACC CGT GAC AAA ATG TGT TCC ATG GTC AAA AAA TGG CAG ACA          390
Asp Leu Thr Arg Asp Lys Met Cys Ser Met Val Lys Lys Trp Gln Thr
            105                       110                       115

ATG ATT GAA GCT CAC GTT GAT GTC AAG ACT ACC GAT GGT TAC TTG CTT          438
Met Ile Glu Ala His Val Asp Val Lys Thr Thr Asp Gly Tyr Leu Leu
120                       125                       130                       135

CGT CTG TTC TGT GTT GGT TTT ACT AAA AAA CGC AAC AAT CAG ATA CGG          486
Arg Leu Phe Cys Val Gly Phe Thr Lys Lys Arg Asn Asn Gln Ile Arg
                    140                       145                       150

AAG ACC TCT TAT GCT CAG CAC CAA CAG GTC CGC CAA ATC CGG AAG AAG          534
Lys Thr Ser Tyr Ala Gln His Gln Gln Val Arg Gln Ile Arg Lys Lys
                155                       160                       165

ATG ATG GAA ATC ATG ACC CGA GAG GTG CAG ACA AAT GAC TTG AAA GAA          582
Met Met Glu Ile Met Thr Arg Glu Val Gln Thr Asn Asp Leu Lys Glu
            170                       175                       180

GTG GTC AAT AAA TTG ATT CCA GAC AGC ATT GGA AAA GGC ATA GAA AAG          630
Val Val Asn Lys Leu Ile Pro Asp Ser Ile Gly Lys Gly Ile Glu Lys
            185                       190                       195

GCT TGC CAA TCT ATT TAT CCT CTC CAT GAT GTC TTC GTT AGA AAA GTA          678
Ala Cys Gln Ser Ile Tyr Pro Leu His Asp Val Phe Val Arg Lys Val
200                       205                       210                       215

AAA ATG CTG AAG AAG CCC AAG TTT GAA TTG GGA AAG CTC ATG GAG CTT          726
Lys Met Leu Lys Lys Pro Lys Phe Glu Leu Gly Lys Leu Met Glu Leu
                    220                       225                       230

CAT GGT GAA GGC AGT AGT TCT GGA AAA GCC ACT GGG GAC GAG ACA GGT          774
His Gly Glu Gly Ser Ser Ser Gly Lys Ala Thr Gly Asp Glu Thr Gly
                235                       240                       245
```

```
GCT AAA GTT GAA CGA GCT GAT GGA TAT GAA CCA CCA GTC CAA GAA TCT          822
Ala Lys Val Glu Arg Ala Asp Gly Tyr Glu Pro Pro Val Gln Glu Ser
        250                     255                 260

GTT TAAAGTTCAG ACTTCAAATA GTGGCAAATA AAAAGTGCTA TTTGTGAAAA              875
Val


AAAAAAAAAA AAAAAAAAAA AAA                                                898


(2) INFORMATION FOR SEQ ID NO:4:

        (i) SEQUENCE CHARACTERISTICS:
            (A) LENGTH: 15 base pairs
            (B) TYPE: nucleic acid
            (C) STRANDEDNESS: single
            (D) TOPOLOGY: linear

        (ii) MOLECULE TYPE: CDS




        (xi) SEQUENCE DESCRIPTION: SEQ ID NO:4:

CTTGCCAACC GCCAT                                                          15

(2) INFORMATION FOR SEQ ID NO:5:

        (i) SEQUENCE CHARACTERISTICS:
            (A) LENGTH: 15 base pairs
            (B) TYPE: nucleic acid
            (C) STRANDEDNESS: single
            (D) TOPOLOGY: linear

        (ii) MOLECULE TYPE: CDS




        (xi) SEQUENCE DESCRIPTION: SEQ ID NO:5:

TCCAAGCCTT ACGCC                                                         15
```